# EUROPEAN PATENT SPECIFICATION

(11) **EP 3 432 868 B1**
(45) Date of publication and mention of the grant of the patent: **16.12.2020**
(21) Application number: 17771049.8
(22) Date of filing: 22.03.2017
(51) Int. Cl.: A61K 9/51, A61K 48/00, A61K 31/522

(54) **THIN-SHELL POLYMERIC NANOPARTICLES AND USES THEREOF**
DÜNNSCHALIGE POLYMERE NANOPARTIKEL UND VERWENDUNGEN DAVON
NANOPARTICULES POLYMÈRES À ENVELOPPE MINCE ET LEURS UTILISATIONS

(30) Priority: 23.03.2016 US 201662312015 P
(43) Date of publication of application: 30.01.2019
(73) Proprietor: Academia Sinica, Taipei, Taiwan (CN)
(72) Inventor: HU, Che-Ming, Jack, Taipei City (TW); CHEN, Hui-Wen, Taipei City (TW); YAO, Bing-Yu, New Taipei City 220 (TW)
(74) Representative: Cabinet Becker et Associés
(86) International application number: PCT/US2017/023566
(87) International publication number: WO 2017/165506

(56) References cited:
- WO-A1-99/58112
- WO-A1-2015/058111
- WO-A1-2015/058111
- US-A1- 2009 028 797
- US-A1- 2010 068 286
- US-A1- 2011 038 939
- US-A1- 2013 209 566
- US-A1- 2013 209 566
- WU, B. et al.: "BIODEGRADABLE COLLOIDAL CARRIERS IN DRUG DELIVERY APPLICATIONS", POLYMERIC DRUG DELIVERY TECHNIQUES, 2015, pages 8-13, XP055578526,

## Description

### BACKGROUND

Polymeric nanoparticles have broad applications as carriers of active agents, i.e., cargoes, in numerous fields such as drug delivery. Yet, the difficulty in creating nanoparticles with large aqueous interiors significantly limits their applications involving encapsulation of hydrophilic macromolecular cargoes.

Existing polymeric nanoparticles are generally solid with little or no internal aqueous core space for hydrophilic cargo encapsulation. Several hollow polymeric nanoparticles consisting of polymeric shells have been developed, but they have various drawbacks, e.g., size limitation, undesired shell thickness and strength, and poor cargo encapsulation efficiency.

In the prior art document US 2013/209566 relates to a novel nanoparticle composition and methods to make a nanoparticle-based drug delivery system. More particularly, the present invention relates to a nanoparticle-based drug delivery system generated from poly(ortho ester) polymers which have sustained drug release capability for the treatment of intraocular diseases.

There is a need to develop a new carrier for delivering bioactive agents without the above-described drawbacks.

### SUMMARY

The present invention as disclosed in claim 1, relates to polymeric nanoparticles for encapsulating hydrophilic bioactive agents. Unexpectedly, polymeric nanoparticles of this invention demonstrate high encapsulation efficiency for hydrophilic bioactive agents with high loadings.

One aspect of this invention is a polymeric nanoparticle for encapsulating a bioactive agent. The polymeric nanoparticle includes (i) a polymeric shell impermeable to water and (ii) one or more aqueous cores enclosed by the polymeric shell and containing the bioactive agent. The polymeric shell has a thickness of 8 - 20 nm and the polymeric nanoparticle has an outer diameter of 100 - 600 nm.

In one example, the polymeric nanoparticle has an outer diameter greater than 100 nm and the aqueous core has a diameter greater than 70% (e.g., > 80%) that of the outer diameter of the polymeric nanoparticle.

Generally, the polymeric nanoparticle has an osmotic resistance of 840 mOsm/kg or higher.

The polymeric nanoparticle of this invention can be used to encapsulate various hydrophilic bioactive agents. Examples of a bioactive agent include a small molecule, a peptide, a protein, a nucleic acid (e.g., siRNA or cyclic di-GMP), an imaging agent, an inorganic nanoparticle, an organic nanoparticle, and a combination thereof. The bioactive agent can have encapsulation efficiency greater than 20% (e.g., > 30% and >40%).

Also within the scope of this invention is a method of treating a disease. The method includes administering to a subject in need thereof the claimed polymeric nanoparticle that encapsulates a bioactive agent for treating the disease.

Further covered by this invention is method of preparing as disclosed in claim 12, the polymeric nanoparticle described above. The method includes the following steps: (i) dissolving a polymer in a solvent to form a polymer solution, (ii) emulsifying by dispersion the polymer solution in a first aqueous solution that contains a bioactive agent to form an emulsion, (iii) emulsifying by fluidic dispersion the emulsion thus formed in a second aqueous solution to obtain a polymeric nanoparticle, and (iv) collecting the polymeric nanoparticle thus obtained. It is important that the polymer contains a non-polar segment and a polar terminal group. Also, the fluidic dispersion is conducted in a controlled manner by using a microfluidizer.

Typically, the solvent used in the above preparation method is a non-polar solvent. Examples of the solvent include, but are not limited to, dichloromethane, benzyl alcohol, ethyl acetate, chloroform, and a mixture containing any molar ratio of the aforementioned solvents.

Each of the first and the second aqueous solutions can be a polar solution that contains a solubilized molecule to modulate the solution's acidity and viscosity, i.e., a modulator. Examples of the modulator include, but are not limited to, sodium phosphate, sodium bicarbonate, Tris-HCl, sucrose, dextran, and a combination thereof.

The details of the invention are set forth in the description below. Other features, objects, and advantages of the invention will be apparent from the following drawings and detailed description of several embodiments, and also from the appending claims.

### BRIEF DESCRIPTION OF THE DRAWINGS

Fig. 1 is a depiction of encapsulation efficiency of polymeric nanoparticles for a nucleic acid and a protein.
Fig. 2 is a depiction of cell uptake and green fluorescent protein (GFP) knockdown with siRNA-GFP.
Fig. 3 is a depiction of the effect of polymeric nanoparticles on encapsulation and controlled release of stimulator of interferon gene (STING) agonists for immune stimulation.
Fig. 4 is a depiction of STING agonist-loaded nanoparticles on enhancing lymphatic cytokines while minimizing systemic cytokines.
Fig. 5 is a depiction of preparing a nanoparticle vaccine via antigen/nanoparticle coupling.
Fig. 6 is a depiction of evaluating the nanoparticle vaccine thus prepared.
Fig. 7 is a depiction of evaluating the nanoparticle vaccine's effect on cellular immune response.
Fig. 8 is a depiction of thin-shell polymeric nanoparticles containing multiple aqueous cores loaded with bioactive agents.

### DETAILED DESCRIPTION

Disclosed in detail herein is a polymeric nanoparticle for encapsulating a hydrophilic bioactive agent, e.g., a therapeutic or vaccine.

Medicinal and vaccine nanotechnology has made a significant impact on the development of novel therapeutics and vaccine formulations. In pharmaceutics development, nanocarriers enable precision drug delivery that improves drugs' therapeutic index, reduce side effects, and promote multidrug synergism. In vaccine development, nanoparticles can enhance the potency of antigenic targets by improving their lymphatic transport, enabling multivalent antigen presentation, and facilitating antigen/adjuvant association. Despite extensive nanoparticle research, a significant challenge remains in encapsulating hydrophilic and macromolecular cargoes. This invention is drawn to a polymeric nanoparticle capable of encapsulating a bioactive agent, e.g., a hydrophilic and macromolecular cargo.

The claimed nanoparticle contains a thin polymeric shell and one or more aqueous cores enclosed by the polymeric shell.

The polymeric shell is formed of an amphiphilic polymer that contains a non-polar segment and a polar terminal group. Examples of the non-polar segment include, but are not limited to, poly(lactic acid), poly(lactic-co-glycolic acid) (PLGA), polycaprolactone, and polyurethane. The PLGA can have any lactic acid to glycolic acid molar ratio (e.g., 50:50 or 75:25 PLGA). The polar terminal group can be a negatively charged group, a positively charged group, a zwitterionic group, or a neutral group. Examples of the negatively charged group include a carboxylic acid, a succinic acid, and a sulfonic acid. Examples of the positively charged group include an amine and an amidine. Examples of the zwitterionic group include a carboxybetaine and a sulfobetaine. An example of the neutral group is a saccharide.

An exemplary polymeric shell is formed of a polymer containing poly(lactic-co-glycolic acid) as the non-polar segment and a carboxylic acid as the polar terminal group.

The polymeric nanoparticle described herein can be a polymeric hollow nanoparticle platform with a defect-free polymeric shell having a thickness of: 8-20 nm The hollow polymeric nanoparticle typically has an outer diameter between 100 and 600 nm.

By minimizing the shell thickness, the polymeric nanoparticle can be formed with a large interior aqueous space capable of maximizing the cargo loading. For particles above 100 nm in outer diameter, the interior aqueous space can possess a diameter at least 80% of the particle's outer diameter or can be of multiple compartments with a large collective volume. High efficiency encapsulation of hydrophilic dyes and nucleic acids are demonstrated with the thin-shell hollow nanoparticles in the absence of complementary binding molecules. The thin-shell nanoparticles are demonstrated to be resistant to osmotic stress, a feature attributable to complete, defect-free polymeric shell that is impermeable to water. The polymeric nanoparticle of this invention can be used for delivering bioactive agents in various fields, including drug delivery and vaccine development.

Polymeric nanoparticles, particularly those consisting of biodegradable and biocompatible polymers such as poly(lactic-co-glycolic acid)(PLGA), have received considerable attention in nanomedicine research because of the polymer's numerous features including biocompatibility, biodegradability, and synthetic flexibility. However, due to the polymer's inherent hydrophobicity, PLGA-based nanoparticles have been limited to the delivery of water-insoluble compounds in clinical. Encapsulation of hydrophilic and macromolecular cargoes in polymeric nanoparticles remains a challenge as polymers tend to form solid nanospheres with little or no aqueous core space to carry hydrophilic and macromolecular cargoes, e.g., siRNA.

Given that macromolecular encapsulation is common in natural nanoparticulates in the form of viruses, it can be envisioned that an ideal nanocarrier should possess a thin shell enclosing a large aqueous volume for the packaging of bioactive molecules. The thin shell is also preferably defect free and water impermeable to allow reliable cargo encapsulation.

Also covered by this invention is a method of using the polymeric nanoparticle described above for treating a medical condition. Examples of the medial condition include, but are not limited to, cardiovascular disease, cancer, autoimmune disease, or infection.

Further disclosed in detail herein is a method of preparing the above-described polymeric nanoparticle.

The thin-shell hollow nanoparticle is prepared based on a double emulsion process using amphiphilic polymers with high contrast of polarity at their terminus. More specifically, a solution of carboxyl-terminated PLGA in dichloromethane (DCM) is first used to emulsify an aqueous phase containing a cargo under sonic dispersion to form an emulsion. The emulsion thus formed is subsequently emulsified in an outer aqueous phase using fluidic dispersion.

By adjusting the polymer concentration and dispersion force or using polymers with defined length and sharp polarity in the double emulsion process, the preparation method described above can provide hollow polymeric nanoparticles with outer diameters between 100-600 nm.

The nanoparticles are prepared based on a water-oil-water double emulsion process in which polymers dissolved in a solvent system is first used to emulsify an aqueous phase. The emulsion is subsequently emulsified by a secondary aqueous phase. The inner and outer aqueous phases can be of any polar solution, e.g., water, acetic acid, and ethanol. The aqueous phase contains solubilized molecules to modulate the solution's acidity and viscosity, which include sodium phosphate and sodium bicarbonate. In one embodiment, water is used as an anti-solvent for the nanoparticle preparation.

The water-oil-water double emulsion method described above for preparing the polymeric nanoparticle of this invention has two key features; namely, (i) emulsion between different phases is achieved through polymers with inherently high contrast in polarity (PLGA with a carboxyl-terminal group) rather than using an surfactant, e.g., vitamin E-D-α-tocopherol polyethylene glycol succinate and poly(vinyl alcohol), which enhances the emulsifying capability to minimize polymer shell thickness and has a higher commercial value without using surfactant materials; and (ii) controlled fluidic dispersion using either a microfluidizer or sonication for the second emulsion process to balance homogenization of the oil phase and retention of encapsulated cargo in the inner aqueous phase.

The polymeric nanoparticle prepared by the above-described method serves as a platform technology for drug delivery, theranostics, and vaccine development applications. It can facilitate delivery of a large class of bioactive agents, including small molecules, peptides, nucleic acids, and proteins, to enhance their therapeutic potency. The thin-shell polymeric hollow nanoparticles can be used to encapsulate bioactive agents, including but not limited to small molecules, peptides, proteins, nucleic acids, imaging agents, inorganic nanoparticles, organic nanoparticles, and any combination of the above. The surface of the platform can be optionally decorated with functional moieties, including small molecules, peptides, proteins, nucleic acids, imaging agents, nanoparticles, for different applications such as long-circulating drug delivery, targeted drug delivery, and antigen delivery.

### Preparation of polymeric nanoparticles

Thin-shell polymeric nanoparticles were produced according to a protocol including the following steps:
1. preparing 10 mg/mL carboxy-terminated PLGA polymers in DCM.
2. emulsifying 50 uL of inner aqueous phase containing bioactive agents in 500 uL of PLGA/DCM solution to form a first emulsion. Probe sonicate continuously at 50% sonication amplitude for 30 seconds.
3. emulsifying the first emulsion in 5 mL of aqueous solution and disperse the mixture under controlled fluidic shear using a microfluidizer to form a second emulsion.
4. adding an additional 30 mL of aqueous solution to the second emulsion and evaporate the solvent at 35 °C.
5. evaporating the DCM in a fume hood for 3 hours to afford a solution.
6. isolating particles from the solution by an ultracentrifuge at 22 kG for 35 min.
7. re-dispersing the particles in a desired solution.

### Characterization and encapsulation of polymeric nanoparticles

By employing the steps described above, hollow polymeric nanoparticles with an average diameter of 110.9 nm were prepared. Statistical average of the particles' shell thickness was derived based on parameters obtained by nanoparticle tracking analysis. Based on the total polymer weight, PLGA density, and the number of resulting nanoparticles, it was calculated that the nanoparticles have a statistical average of 16.5 nm in shell thickness. Unexpectedly, certain polymeric nanoparticles had diameters less than 40 nm.

The thin-shell hollow nanoparticles were found to be osmotically resistant resulting from the water impermeable polymeric shells. In a test, 100 nm hollow nanoparticles encapsulating a hydrophilic red food coloring were suspended in solutions ranging from water to 3X PBS, the difference in osmolarity (between 0 to 850 Osmo/kg) did not cause the hollow nanoparticles to release their cargoes. Following 10 min of incubation in their respective solutions, nanoparticles were pelleted under centrifugation at 30,000 g for 5 min, and the resulting pellets showed similar, reddish color indicating retention of hydrophilic dye in the particles. Detection of the supernatant for the released dye based on an absorbance method showed no detectable signals. The study shows that despite having a thin polymeric shell below 20 nm, the hollow nanoparticles had defect-free shells that made them resistant to osmotic stress.

To further demonstrate that the shell of the thin-shell hollow nanoparticles were solid rather than fluid, hollow nanoparticles were subjected to mechanical stress to break the shell. In a cryo-EM visualization, a broken hollow nanoparticle was observed. The observed image of the broken hollow nanoparticles was indicative of a hollow sphere with a solid shell, in contrast to the polymeric vesicles that undergo vesicular reorganization upon mechanical perturbation. The solid polymeric shell led to the water impermeability and osmotic resistance that were not observed in known hollow nanostructures.

A distinguishing feature of the thin-shell polymeric nanoparticle platform is its capacity to encapsulate a large amount of hydrophilic cargoes with its large interior aqueous space. The thin-shell hollow nanoparticles were subjected to encapsulate several bioactive agents, including siRNA and an immunological adjuvant cyclic di-GMP. Unexpectedly, high encapsulation efficiency was achieved for hydrophilic contents of various length scales, including small molecules (e.g., sulfo-cy5, cyclic di-GMP, and cyclic cGAMP), peptides (e.g., ovalbumin peptide OTI (SIINFEKL) or OTII (AAHAEINEA)), nucleic acids (e.g., CpG-oligodeoxynucleotides, 20-mer single standed DNA, and 20-mer siRNA), and proteins (e.g., bovine serum albumin (BSA) and CRISPR-Cas9 nuclease), which were successfully encapsulated with an efficiency above 30% within the compounds' solubility limits. For example, siRNA was encapsulated at an efficiency of 50% with a final loading yield of about 1 nmol per mg of nanoparticles and cyclic di-GMP was encapsulated at a 37% loading efficiency. Silencing of a green fluorescent protein (GFP) gene in GFP-expressing HeLa cells was observed using siRNA loaded thin-shell hollow nanoparticles.

### EXAMPLE 1: Encapsulation of polymeric nanoparticles with hydrophilic macromolecules

An assay was performed to evaluate the encapsulation efficiency of polymeric nanoparticles for two hydrophilic macromolecules, i.e., a nucleic acid (dye-labelled 20-mer single stranded DNA) and a protein (dye-labelled BSA).

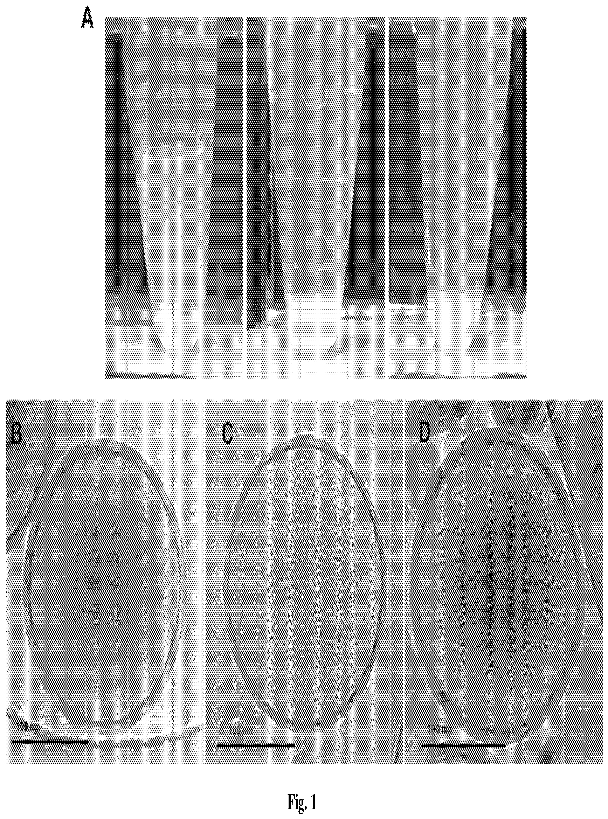

Fig. 1, A-D, is a depiction of encapsulation efficiency of polymeric nanoparticles for a nucleic acid and a protein.
A: Observation of empty nanoparticles (left), nanoparticles loaded with dye-labelled DNA (middle), and nanoparticles loaded with dye-labelled bovine serum albumin (right) following pelleting by ultracentrifugation. Distinctive colored pellets indicate successful encapsulation of DNA and BSA proteins.
B: Empty nanoparticles visualized by cryoEM.
C: DNA-loaded nanoparticles visualized by cryoEM.
D: BSA protein-loaded nanoparticles visualized by cryoEM. Effective DNA and protein loading could be observed through the highly grainy textures inside nanoparticles.

In this study, effective encapsulation of a nucleic acid and a protein was demonstrated using dye-labelled 20-mer single stranded DNA (Fig. 1A; middle) and dye-labelled BSA (Fig. 1A; right). Upon pelleting the nanoparticles, the particle pellets were found to be yellow colored (indicative of the yellowish FAM dye label) as opposed to the white pellet of the empty nanoparticles (Fig. 1A; left). Fluorescence quantification indicates that the nanoparticles unexpectedly exhibited encapsulation efficiencies of 42% and 35% for the nucleic acid and protein, respectively.

Effective encapsulation of the nucleic acid and protein was also visualized using cryoEM. While empty particles showed a plain, even texture in its aqueous core (Fig. 1B), DNA-loaded particles (Fig. 1C) and BSA-loaded particles (Fig. 1D) showed highly grainy textures that are characteristics of concentrated biological contents under cryoEM. Based on the loading concentration of 8 mM DNA and 50 mg/mL BSA for the present embodiments, the DNA-loaded nanoparticle contained about 3500 DNA molecules and the BSA-loaded nanoparticle contains about 260 proteins. Importantly, such high loading of a nucleic acid and a protein have not been previously achieved. Indeed, known PLGA-based nanoformulations consistently showed very poor nucleic acids encapsulation, and polycationic polymers had to be employed to neutralize the negative charges on nucleic acids to enhance loading. For example, see Shi et al., Angew Chem Int Ed Engl, 2011, 50(31): 7027-31; and Woodrow et al., Nature Materials, 2009, 8(6): 526-533.

These results described above indicate that the thin-shell polymeric nanoparticles of this invention unexpectedly exhibited high encapsulation efficiency, i.e., high loading efficiency, for hydrophilic macromolecules in their native, soluble state, highlighting the advantage and uniqueness of the thin-shell hollow nanoparticles.

### EXAMPLE 2: Effect of polymeric nanoparticles on delivery of siRNA to cells for RNA interference

An assay was performed to evaluate the effect of polymeric nanoparticles on delivery of siRNA to cells for RNA interference.

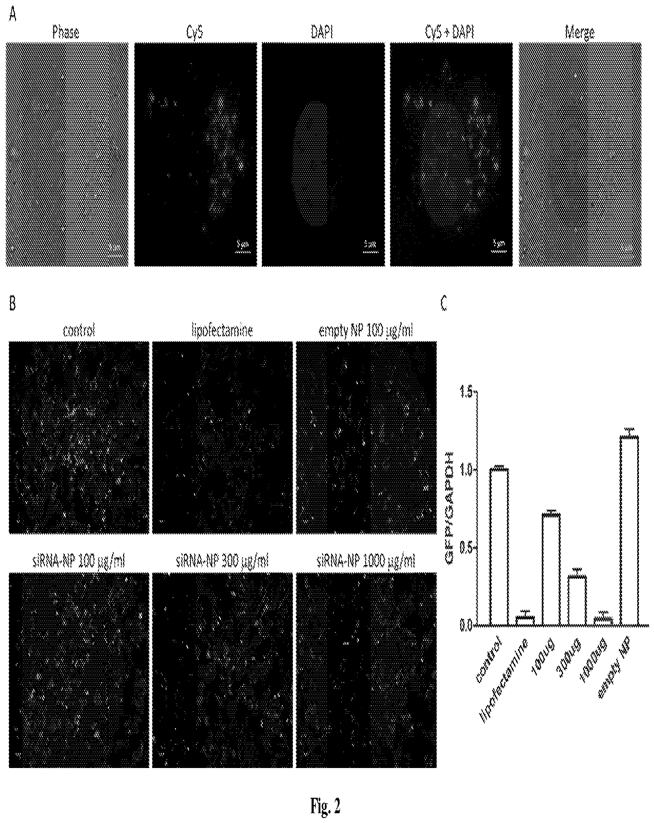

Fig. 2, A-C, is a depiction of cell uptake and GFP knockdown with siRNA-GFP.
A: Hela cell uptake sulfo-Cy5 NP for 24h and nucleus stained with DAPI. Images were taken by confocal microscope.
B: GFP signal in different treated cells. Hela-GFP cellS were treated with siRNA-GFP-NP (100, 300 and 1000 ug/ml PLGA) for 24h. Cells transfected with lipofactamine RNAiMax and cells incubated with empty NP served as controls.
C: Dose response of siRNA-GFP-NP at 24h treatment. RNA was extracted and reverse transcripted to cDNA. GFP mRNA level was measured by qRT-PCR and normalized to glyceraldehyde 3-phosphate dehydrogenase (GAPDH).

In this study, the nanoparticle platform was demonstrated to successfully deliver siRNA to cells for RNA interference. Cell uptake experiment was first performed using sulfo-Cy5-loaded PLGA nanoparticle to track the internalization of nanoparticles in cells. Hela cells were treated with sulfo-Cy5 NP for 24h, nucleus was stained with DAPI and image was taken by confocal microscope. After 24h incubation, sulfo-Cy5 NPs were uptake by cells and accumulated in the cytoplasm (Fig. 2A). Next, whether the siRNA-loaded nanoparticle could specifically knockdown the target gene expression was tested in Hela-GFP cells. siRNA against GFP sequence was encapsulated in thin-shell polymeric nanoparticles and mixed with cells for 24h and 72h. Cells transfected with siRNA by RNAiMax lipofectamine served as positive control. At indicated time point, cells were imaged by fluorescence microscope (Fig. 2B) and then total RNA was extracted by Trizol reagent. RNA was reverse transcripted to cDNA and silencing of GFP was determined by qRT-PCR and normalized to GAPDH. GFP mRNA level was knockdown to 5% after 24h with lipofectamine transfection. Cells treated with 100, 300, and 1000 ug/ml of nanoparticles showed dose-dependent reduction of GFP mRNA level to 70%, 30%, and 4%, respectively (Fig. 2C). High concentration of nanoparticle showed similar knockdown efficiency as the well-known commercial product lipofectamine. Importantly, no cytotoxicity phenomena was observed in cells treated with siRNA-GFP-NP even at the highest concentration of 1000 ug/ml PLGA amount, indicating the superior safety of the platform.

These results indicate that the polymeric nanoparticles exhibited high efficiency on delivery of siRNA to cells for RNA interference

### EXAMPLE 3: Effect of polymeric nanoparticles on encapsulation and controlled release of stimulator of interferon gene (STING) agonists for immune stimulation in lymph nodes

An assay was performed to evaluate the effect of polymeric nanoparticles on encapsulation and controlled release of STING agonists for immune stimulation in lymph nodes.

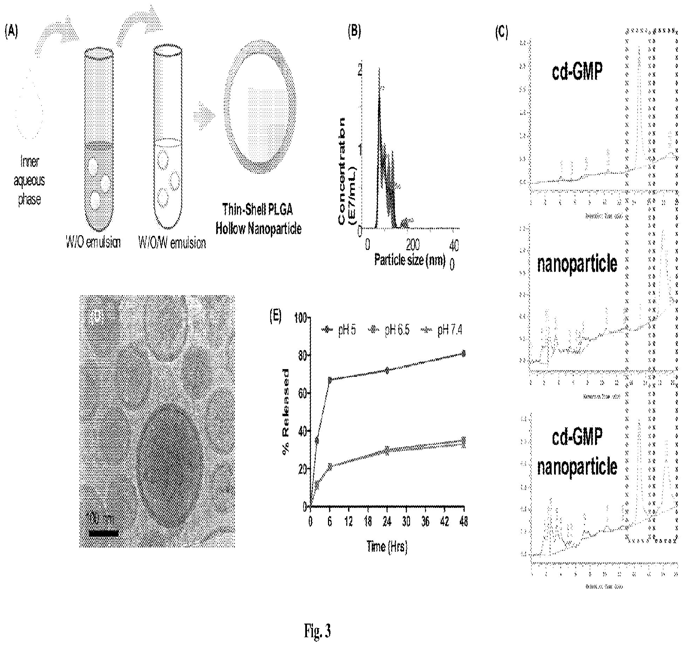

Fig. 3, A-E, is a depiction of the effect of polymeric nanoparticles on encapsulation and controlled release of STING agonists for immune stimulation.
A: Preparation of adjuvant-loaded thin-shell hollow nanoparticles.
B: Size of nanoparticles as measured by nanoparticle tracking analysis.
C: Encapsulation of cyclic-di-GMP as verified by gradient HPLC.
D: CryoEM visualization of thin-shell hollow nanoparticles.
E: Cargo release study revealed a pH-sensitive triggered release profile for the nanoparticles.

In this study, the present platform was applied for vaccine development. A major technical challenge in preparing nanoparticle vaccines lies in reliably associating antigens and adjuvants on a nanoscale substrate. For example, see Brannon-Peppas et al., Adv Drug Deliv Rev, 2004, 56(11): 1649-59; and Lima-Tenorio et al., Int J Pharm, 2015, 493(1-2): 313-27. To overcome this technical challenge, thin-shell polymeric hollow nanoparticles were used to package a high density of functional cargoes using a biodegradable polymer, i.e., PLGA.

More specifically, hollow particles between 100 to 200 nm in diameter were prepared using a double emulsion process (Figs. 3A and 3B). They efficiently encapsulated a STING agonist adjuvant, cyclic di-GMP (cd-GMP), at about 40% efficiency (Fig. 3C), which is notable as nucleic acids and hydrophilic cargoes are notoriously difficult to encapsulate in nanoparticle platforms. The high encapsulation efficiency yielded approximately 2,000 STING agonist molecules per nanoparticle. No nanoformulations of STING agonists based on polymeric nanoparticles have been reported. Examination by cryoEM revealed large interior cores in these hollow nanoparticles, which were responsible for the high loading efficiency of cd-GMP (Fig. 3D). Upon intracellular delivery, the thin polymeric shell of the hollow nanoparticles was triggered by acidic pH to rapidly release the interior content (Fig. 3E).

These results indicate that the thin-shell polymeric nanoparticles enabled effective preparation of synthetic vaccines.

### EXAMPLE 4: Evaluation of STING agonist-loaded nanoparticles on enhancing lymphatic cytokines while minimizing systemic cytokines

An assay was performed to evaluate STING agonist-loaded nanoparticles on enhancing lymphatic cytokines while minimizing systemic cytokines.

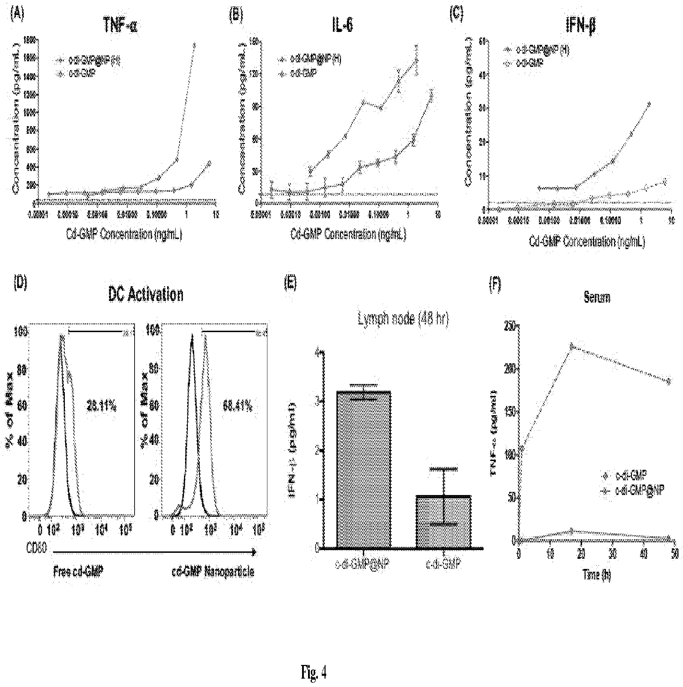

Fig. 4, A-F, is a depiction of STING agonist-loaded nanoparticles on enhancing lymphatic cytokines while minimizing systemic cytokines.
A: Induction of TNF-α in a mouse dendritic cell line following incubation with cd-GMP nanoparticles and free cd-GMP.
B: Induction of IL-6 in the same mouse dendritic cell line.
C: Induction of IFN-β in the mouse dendritic cell line.
D: Activation of JAWSII upon incubation with equivalent doses of cd-GMP in free molecule form and in nanoparticle formulation.
E: Quantification of lymph node IFN-β 48 hours following footpad injection of free cd-GMP or nanoparticle cd-GMP.
F: Level of systemic TNF-α following footpad injection of free cd-GMP and nanoparticle cd-GMP.

Immune activation by the cd-GMP-loaded nanoparticles was first examined *in vitro* using a mouse dendritic cell line, JAWSII. Following 24 hours of incubation with either free cd-GMP or cd-GMP nanoparticles, the culture supernatants were collected for ELISA analysis to quantify the levels of TNF-α, IL-6, and IFN-β. As compared to free cd-GMP formulation, the nanoparticle formulation more effectively triggered the production of TNF-α, IL-6, and IFN-β (Figs. 4A, 4B, and 4C). CD80 expression on the cells was also enhanced when incubated with the nanoparticle formulation as compared to an equivalent dose of free cd-GMP (Fig. 4D). It was observed that the nanoparticles enhanced the adjuvanticity of the STING agonist by about 30 folds, attributable to increased intracellular delivery by the nanocarrier. It can be inferred that the free cyclic-di-nucleotide is not readily membrane permeable and may not easily access its cytosolic target. Upon nanoparticle encapsulation, cellular uptake is enhanced via particle endocytosis and the subsequent intracellular release facilitates cytosolic entry of cd-GMP, thereby enhancing its immune potentiating effect.

Immune potentiation by the cd-GMP nanoparticles was further compared to free cd-GMP *in vivo* in mice. 48 hours following footpad injections, the draining popliteal lymph nodes were collected for IFN-β quantification. It was observed that the nanoparticle formulation induced a significantly higher level of IFN-β in the lymph node (Fig. 4E), which is important for proper T cell maturation. In addition, the systemic level of TNF-α, an indicator of reactogenicity, was also monitored following the footpad administration of free cd-GMP and cd-GMP nanoparticles. It was observed that the nanoparticles resulted in a significantly lower serum level of TNF-α, which reflects the nanoparticles' distribution in the lymphatic system. The free cd-GMP, on the other hand, induced an elevated level of serum TNF-α and these small molecules could freely diffuse into the blood stream (Fig. 4F).

These results indicate that STING agonist-loaded nanoparticles effectively enhanced lymphatic cytokines for lymph node-targeted immune potentiation.

### EXAMPLE 5: Preparation and evaluation of a nanoparticle vaccine

An assay was performed to prepare a nanoparticle vaccine via antigen/nanoparticle coupling and evaluate the nanoparticle vaccine thus prepared.

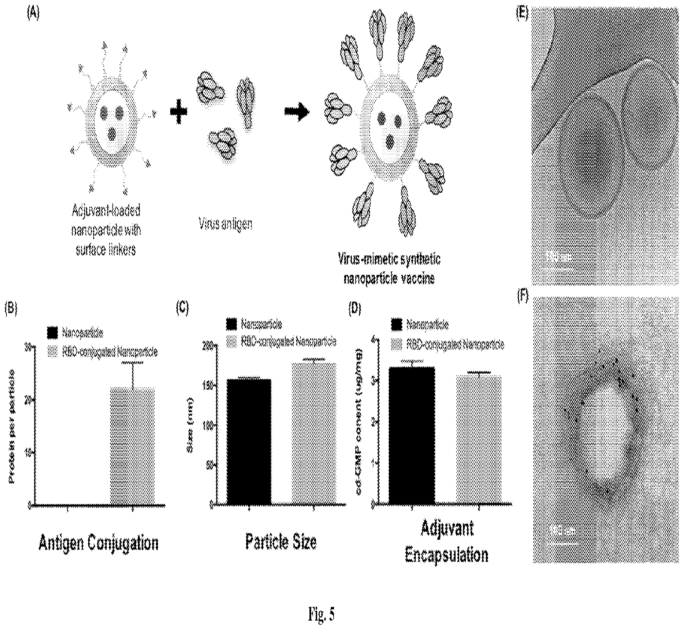

Fig. 5, A-F, is a depiction of preparing a nanoparticle vaccine via antigen/nanoparticle coupling.
A: Preparation of virus-mimetic nanoparticle vaccine via spontaneous linkage between functionalized nanoparticles and antigens.
B: Quantification of antigen content via BCA assay.
C: Nanoparticle size before and after antigen conjugation as measured by dynamic light scattering.
D: Quantification of nanoparticle encapsulated cd-GMP before and after protein conjugation.
E: Cryo-EM visualization of nanoparticles following antigen conjugation.
F: Immunogold staining against viral antigen verifies successful antigen conjugation on the nanoparticles.

Using cdGMP-loaded thin-shell polymeric nanoparticles, a nanoparticle vaccine was prepared for Middle East respiratory syndrome coronavirus (MERS-CoV) with the receptor binding domain (RBD) of MERS-CoV spike proteins. The RBD was expressed in serum free-adapted Sf21 insect cells and confirmed by Western blot using anti-MERS-CoV RBD polyclonal antibody and anti-His antibody. A pure product of the 35 kDa RBD protein can be obtained after purification by Histrap column on a fast protein liquid chromatography. To enable antigen/nanoparticle coupling, cd-GMP-loaded nanoparticles were first prepared with maleimide-terminated surface linkers, which spontaneously formed covalent bonding with available thiol groups (Fig. 5A). The purified RBD proteins were then treated with a mild reducing agent (tris(2-carboxyethyl)phosphine), which reduces disulfide bonds into free thiols. The reduced RBD proteins were then mixed with the maleimide-functionalized nanoparticles for 4 hours under gentle mixing. The RBD-conjugated nanoparticles were isolated from free proteins via centrifugation at 30,000 x g. Upon nanoparticle collection, BCA assay revealed that the resulting nanoparticles contained about 20% of the antigen input, corresponding to about 20 protein antigens per particle (Fig. 5B). Dynamic light scattering showed that the nanoparticles increased in diameter from 150 nm to 179 nm following the protein conjugation (Fig. 5C), indicating successful antigen/particle coupling that increased the particle's overall hydrodynamic size.

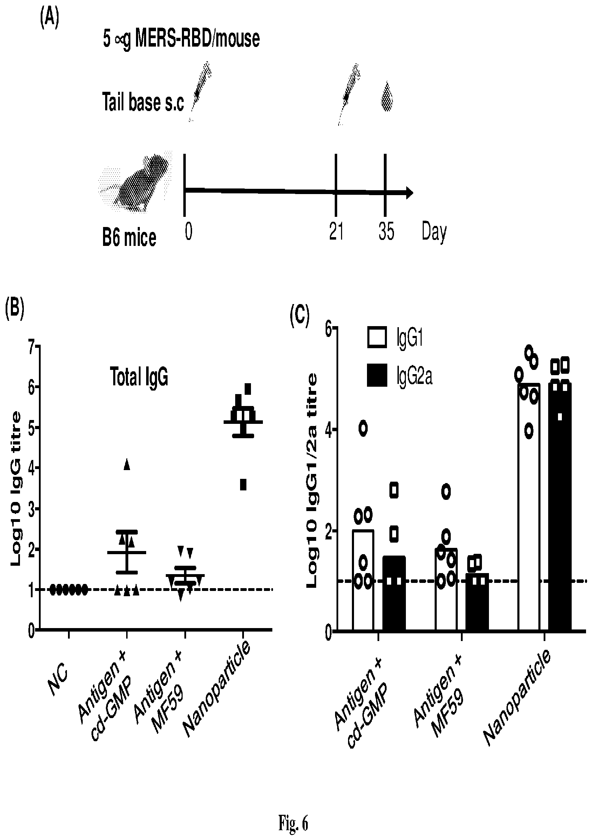

Fig. 6, A-C, is a depiction of evaluating the nanoparticle vaccine described above.
A: Vaccination schedule for the vaccine evaluation.
B: Total anti-RBD IgG titer quantification on day 35, 2 weeks following the booster vaccination.
C: Quantification of anti-RBD IgG1 and IgG2a titers on day 35.

Antigen-specific IgG antibody responses were evaluated and compared with other vaccine nanoparticles, including free antigen mixed with free cd-GMP and free antigen mixed with MF59. Mice were inoculated with the different vaccine nanoparticles described above on day 0 and day 21, and sera of all immunized mice were collected for ELISA analysis on day 35 (Fig. 6A). Unexpectedly, the synthetic nanoparticles induced significantly higher levels of antibody titers among all groups (Fig. 6B). It is noteworthy that, the level of IgG2a, an indicator of Th1 immune response, was also increased following the nanoparticle inoculation (Fig. 6C).

The results demonstrate that the nanoparticle platform enabled preparation of nanoparticle vaccine that exhibited superior advantages in raising humoral responses.

### EXAMPLE 6: Evaluation of nanoparticles co-encapsulating CD8 antigen and STING agonist on inducing CD8 T cell response

An assay was performed to evaluate the effect of polymeric nanoparticles co-encapsulating CD8 antigen (SIINFEKL) and STING agonist on promoting antigen-specific CD8 T cell response.

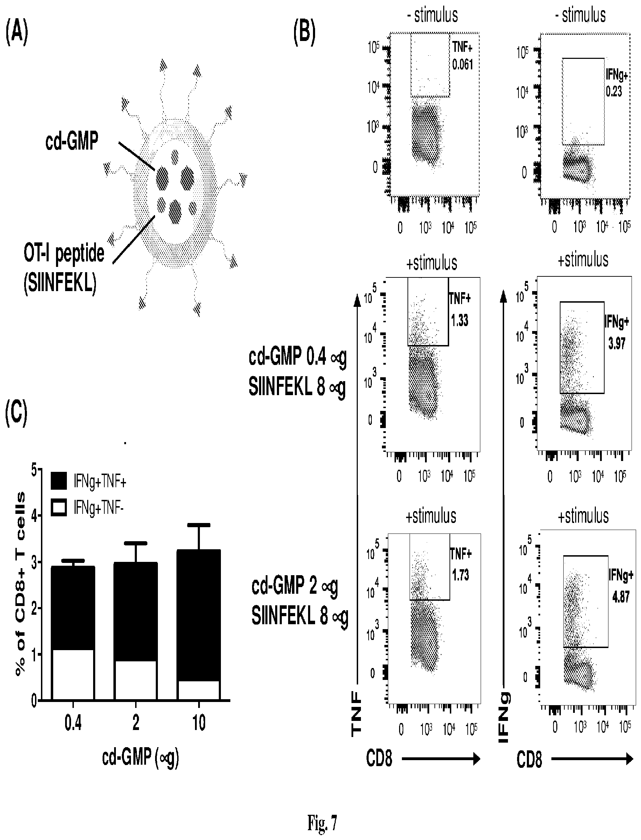

Fig. 7, A-C, is a depiction of the effect of polymeric nanoparticles co-encapsulating SIINFEKL peptide and STING agonist on promoting SIINFEKL-specific CD8 T cell reponse.
A: Nanoparticles co-encapsulating SINNFEKL peptides and STING agonist (cd-GMP).
B: Frequency of SIINFEKL-specific CD8 T cells 7 days following nanoparticle immunization.
C: Quantification of polyfunctional (IFNg+TNF+) CD8+ T cells following immunization with nanoparticles containing different doses of cd-GMP.

To evaluate the antigen-specific cellular immunity induced by the polymeric nanoparticles, 3 C57BL/6 mice were immunized via the subcutaneous route with nanoparticles containing OVA257-264 H2-Kb-restricted peptide SIINFEKL (8 µg per mouse) and different amounts of cd-GMP (0.4, 2, or 10 µg per mouse) (Fig. 7A). The mice were euthanized 7 days after immunization, and the spleens were harvested for analyzing CD8+ T cell responses by intracellular cytokine staining. It was observed that the nanoparticles induced antigen-specific CD8+ T cell cytokine production in a manner dependent on the dose of cd-GMP (Fig. 7B). Furthermore, the mice receiving higher amounts of cd-GMP showed more polyfunctional CD8+ T cell responses (Fig. 7C).

These results indicate that the polymeric nanoparticles of this invention exerted high efficacy in promoting antigen-specific T cell immunity. They also demonstrate that the cargo encapsulation could be modulated by combining peptides and nucleic acids in varying amounts in the nanoparticles.

### EXAMPLE 7: Preparation of thin-shell polymeric nanoparticles with multiple aqueous cores loaded with bioactive agents.

An assay was performed to prepare thin-shell polymeric nanoparticles with multiple aqueous cores loaded with a 20-mer single stranded DNA.

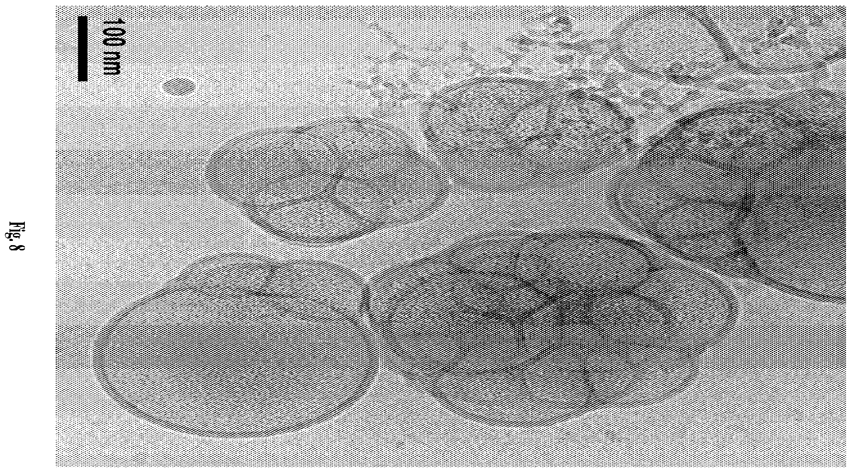

Fig. 8 is a depiction of the thin-shell polymeric nanoparticles with multiple aqueous cores enclosed by a polymeric thin shell. Each core was loaded with DNA showing a dense, grainy texture.

It was found that the number of aqueous cores inside the thin-shell polymeric nanoparticles could be modulated by controlling the extent of dispersion during the formation of the first and second emulsions described above. To prepare nanoparticles containing multiple aqueous cores, the fluid pressure in the microfluidizer was reduced (2000 psi) to afford larger water/oil/water emulsions with multiple aqueous phases per single emulsion droplet. Following solvent evaporation, cryoEM visualization showed thin-shell polymeric nanoparticles containing multiple aqueous cores (Fig. 8). Each aqueous core was enclosed by a polymeric thin shell of below 20 nm in thickness. The cryoEM image of the polymeric nanoparticles encapsulated with DNA revealed a dense grainy texture in each of the aqueous cores, indicating successful cargo encapsulation (Fig. 8).

This result demonstrates that the polymeric nanoparticles of this invention had multiple aqueous cores, each encapsulating a bioactive agent.

## Claims

1. A polymeric nanoparticle for encapsulating a hydrophilic bioactive agent, the polymeric nanoparticle comprising:
a polymeric shell impermeable to water, and
one or more aqueous cores enclosed by the polymeric shell, the one or more aqueous cores each containing the hydrophilic bioactive agent,
wherein the polymeric shell is formed of a polymer containing a non-polar segment and a polar terminal group and has a thickness of 8-20 nm, and the polymeric nanoparticle has an outer diameter of 100-600 nm.

2. The polymeric nanoparticle of claim 1, wherein the aqueous core has a diameter greater than 70% that of the outer diameter of the polymeric nanoparticle.

3. The polymeric nanoparticle of claim 2, wherein the aqueous core has a diameter greater than 80% that of the outer diameter of the polymeric nanoparticle.

4. The polymeric nanoparticle of claim 1, wherein the non-polar segment is poly(lactic acid), poly(lactic-co-glycolic acid), polycaprolactone, or polyurethane.

5. The polymeric nanoparticle of claim 1, wherein the polar terminal group is i) a negatively charged group being a carboxylic acid, a succinic acid, or a sulfonic acid ii) a positively charged group being an amine or an amidine, iii) a zwitterionic group being a carboxybetaine or a sulfobetaine, or iv) a neutral group being a saccharide.

6. The polymeric nanoparticle of claim 1, wherein the non-polar segment is poly(lactic-co-glycolic acid) and the polar terminal group is a carboxylic acid.

7. The polymeric nanoparticle of claim 1, wherein the polymeric nanoparticle has an osmotic resistance of 840 mOsm/kg or higher.

8. The polymeric nanoparticle of claim 1, wherein the hydrophilic bioactive agent is selected from the group consisting of a small molecule, a peptide, a protein, a nucleic acid, an imaging agent, an inorganic nanoparticle, an organic nanoparticle, and a combination thereof.

9. The polymeric nanoparticle of claim 8, wherein the bioactive agent has an encapsulation efficiency greater than 20%.

10. The polymeric nanoparticle of claim 8, wherein the hydrophilic bioactive agent is siRNA or cyclic di-GMP

11. A polymeric nanoparticle encapsulating a hydrophilic bioactive agent as defined in any of claims 1 to 10, for use in treating a disease in a subject, wherein the disease is cardiovascular disease, cancer, autoimmune disease, or infection.

12. A method of preparing the polymeric nanoparticle of claim 1, comprising:
dissolving a polymer in a solvent to form a polymer solution, emulsifying by dispersion the polymer solution in a first aqueous solution that contains a hydrophilic bioactive agent to form an emulsion,
emulsifying by fluidic dispersion the emulsion thus formed in a second aqueous solution to obtain a polymeric nanoparticle, and
collecting the polymeric nanoparticle thus obtained,
wherein the polymer contains a non-polar segment and a polar terminal group, and the fluidic dispersion is conducted in a controlled manner by using a microfluidizer.

13. The method of claim 12, wherein the solvent is a non-polar solvent selected from the group consisting of dichloromethane, benzyl alcohol, ethyl acetate, chloroform, and a combination thereof.

14. The method of claim 12 or 13, wherein each of the first and the second aqueous solutions contains a modulator selected from the group consisting of sodium phosphate, sodium bicarbonate, Tris-HCl, sucrose, dextran, and a combination thereof.

## Patentansprüche

1. Ein polymeres Nanopartikel zum Verkapseln eines hydrophilen bioaktiven Mittels, wobei das polymere Nanopartikel umfasst:
eine polymere Schale, die für Wasser undurchlässig ist, und
einen oder mehrere wässrige Kerne, die von der polymeren Schale eingeschlossen sind, wobei der eine oder die mehreren Kerne jeweils das hydrophile bioaktive Mittel enthalten,
wobei die polymere Schale aus einem Polymer gebildet ist, das ein unpolares Segment und eine polare Endgruppe enthält, und eine Dicke von 8-20 nm besitzt, und das polymere Nanopartikel einen Außendurchmesser von 100-600 nm besitzt.

2. Das polymere Nanopartikel nach Anspruch 1, wobei der wässrige Kern einen Durchmesser besitzt, der mehr als 70% des Außendurchmessers des polymeren Nanopartikels beträgt.

3. Das polymere Nanopartikel nach Anspruch 2, wobei der wässrige Kern einen Durchmesser besitzt, der mehr als 80% des Außendurchmessers des polymeren Nanopartikels beträgt.

4. Das polymere Nanopartikel nach Anspruch 1, wobei das unpolare Segment Poly(milchsäure), Poly(milch-co-glycolsäure), Polycaprolacton oder Polyurethan ist.

5. Das polymere Nanopartikel nach Anspruch 1, wobei die polare Endgruppe ist i) eine negativ geladene Gruppe, die eine Carbonsäure, eine Succinsäure oder eine Sulfonsäure ist, ii) eine positiv geladenen Gruppe, die ein Amin oder ein Amidin ist, iii) eine zwitterionische Gruppe, die ein Carboxybetain oder ein Sulfobetain ist, oder iv) eine neutrale Gruppe, die ein Saccharid ist.

6. Das polymere Nanopartikel nach Anspruch 1, wobei das unpolare Segment Poly(milch-co-glycolsäure) ist und die polare Endgruppe eine Carbonsäure ist.

7. Das polymere Nanopartikel nach Anspruch 1, wobei das polymere Nanopartikel einen osmotischen Widerstand von 840 mOsm/kg oder höher besitzt.

8. Das polymere Nanopartikel nach Anspruch 1, wobei das hydrophile bioaktive Mittel aus der Gruppe ausgewählt ist, bestehend aus einem kleinen Molekül, einem Peptid, einem Protein, einer Nukleinsäure, einem bildgebenden Mittel, einem anorganischen Nanopartikel, einem organischen Nanopartikel und einer Kombination davon.

9. Das polymere Nanopartikel nach Anspruch 8, wobei das bioaktive Mittel eine Verkapselungseffizienz größer als 20% besitzt.

10. Das polymere Nanopartikel nach Anspruch 8, wobei das hydrophile bioaktive Mittel siRNA oder cyclisches Di-GMP ist.

11. Ein polymeres Nanopartikel, das ein wie in einem der Ansprüche 1 bis 10 definiertes hydrophiles bioaktives Mittel verkapselt, zur Verwendung in der Behandlung in einer Person, wobei die Krankheit eine kardiovaskuläre Krankheit, ein Krebs, eine Autoimmunkrankheit oder eine Infektion ist.

12. Ein Verfahren zur Herstellung des polymeren Nanopartikels nach Anspruch 1, umfassend Lösen eines Polymers in einem Lösemittel, um eine Polymerlösung zu bilden, Emulgieren mittels Dispersion der Polymerlösung in einer ersten wässrigen Lösung, die ein hydrophiles bioaktives Mittel enthält, um eine Emulsion zu bilden,
Emulgieren mittels fluider Dispersion der auf diese Weise gebildeten Emulsion in einer zweiten wässrigen Lösung, um ein polymeres Nanopartikel zu erhalten, und
Sammeln des auf diese Weise erhaltenen polymeren Nanopartikels,
wobei das Polymer ein unpolares Segment und eine polare Endgruppe enthält, die fluide Dispersion in kontrollierter Weise unter Verwendung eines Microfluidizers durchgeführt wird.

13. Das Verfahren nach Anspruch 12, wobei das Lösemittel ein unpolares Lösemittel ist, ausgewählt aus der Gruppe, bestehend aus Dichlormethan, Benzylalkohol, Ethylacetat, Chloroform und einer Kombination davon.

14. Das Verfahren nach Anspruch 12 oder 13, wobei die erste als auch die zweite wässrige Lösung jeweils einen Modulator enthält, ausgewählt aus der Gruppe, bestehend aus Natriumphosphat, Natriumbicarbonat, Tris-HCl, Sucrose, Dextran und einer Kombination davon.

## Revendications

1. Nanoparticule polymère pour encapsuler un agent bioactif hydrophile, la nanoparticule polymère comprenant :
une gaine polymère imperméable à l'eau, et
un ou plusieurs cœurs aqueux enfermés par la gaine polymère, chacun du ou des cœurs aqueux contenant l'agent bioactif hydrophile,
dans laquelle la gaine polymère est formée d'un polymère contenant un segment non polaire et un groupe terminal polaire et a une épaisseur de 8 à 20 nm, et laquelle nanoparticule polymère a un diamètre extérieur de 100 à 600 nm.

2. Nanoparticule polymère selon la revendication 1, dans laquelle le cœur aqueux a un diamètre supérieur à 70 % du diamètre extérieur de la nanoparticule polymère.

3. Nanoparticule polymère selon la revendication 2, dans laquelle le cœur aqueux a un diamètre supérieur à 80 % du diamètre extérieur de la nanoparticule polymère.

4. Nanoparticule polymère selon la revendication 1, dans laquelle le segment non polaire est de l'acide polylactique, de l'acide poly(lactique-co-glycolique), de la polycaprolactone, ou du polyuréthane.

5. Nanoparticule polymère selon la revendication 1, dans laquelle le groupe terminal polaire est i) un groupe chargé négativement qui est un acide carboxylique, un acide succinique ou un acide sulfonique, ii) un groupe chargé positivement qui est une amine ou amidine, iii) un groupe zwittérionique qui est une carboxybétaïne ou une sulfobétaïne, ou iv) un groupe neutre qui est un saccharide.

6. Nanoparticule polymère selon la revendication 1, dans laquelle le segment non polaire est l'acide poly(lactique-co-glycolique), et le groupe terminal polaire est un acide carboxylique.

7. Nanoparticule polymère selon la revendication 1, laquelle nanoparticule polymère a une résistance osmotique de 840 mOsm/kg ou plus.

8. Nanoparticule polymère selon la revendication 1, dans laquelle l'agent bioactif hydrophile est choisi dans l'ensemble constitué par une petite molécule, un peptide, une protéine, un acide nucléique, un agent d'imagerie, une nanoparticule inorganique, une nanoparticule organique, et une combinaison de ceux-ci.

9. Nanoparticule polymère selon la revendication 8, dans laquelle l'agent bioactif a une efficacité d'encapsulation supérieure à 20 %.

10. Nanoparticule polymère selon la revendication 8, dans laquelle l'agent bioactif hydrophile est un ARNsi ou un di-GMP cyclique.

11. Nanoparticule polymère encapsulant un agent bioactif hydrophile telle que définie dans l'une quelconque des revendications 1 à 10, pour une utilisation dans le traitement d'une maladie chez un sujet, dans laquelle la maladie est une maladie cardiovasculaire, un cancer, une maladie auto-immune, ou une infection.

12. Méthode de préparation de la nanoparticule polymère de la revendication 1, comprenant :
la dissolution d'un polymère dans un solvant pour former une solution de polymère, l'émulsification par dispersion de la solution de polymère dans une première solution aqueuse qui contient un agent bioactif hydrophile pour former une émulsion,
l'émulsification par dispersion fluidique de l'émulsion ainsi formée dans une deuxième solution aqueuse pour que soit obtenue une nanoparticule polymère, et
la collecte de la nanoparticule polymère ainsi obtenue,
dans laquelle le polymère contient un segment non polaire et un groupe terminal polaire, la dispersion fluidique est effectuée d'une manière contrôlée par utilisation d'un microfluidiseur.

13. Méthode selon la revendication 12, dans laquelle le solvant est un solvant non polaire choisi dans l'ensemble constitué par le dichlorométhane, l'alcool benzylique, l'acétate d'éthyle, le chloroforme, et une combinaison de ceux-ci.

14. Méthode selon la revendication 12 ou 13, dans laquelle chacune des première et deuxième solutions aqueuses contient un modulateur choisi dans l'ensemble constitué par le phosphate de sodium, le bicarbonate de sodium, le tris-HCl, le saccharose, le dextrane, et une combinaison de ceux-ci.
